## Europäisches Patentamt
## European Patent Office
## Office européen des brevets

(19)

(11) Publication number: **0 016 482**
**B1**

(12) **EUROPEAN PATENT SPECIFICATION**

(45) Date of publication of patent specification: **09.02.83**

(51) Int. Cl.³: **C 07 C 121/24,**
**C 07 C 120/02**

(21) Application number: **80200143.8**

(22) Date of filing: **20.02.80**

(54) Process for the preparation of succinonitrile.

(30) Priority: **01.03.79 NL 7901626**

(43) Date of publication of application:
**01.10.80 Bulletin 80/20**

(45) Publication of the grant of the patent:
**09.02.83 Bulletin 83/6**

(84) Designated Contracting States:
**BE DE FR GB IT NL**

(56) References cited:
**DE - A - 957 390**
**DE - A - 2 719 867**
**DE - B - 1 007 313**
**US - A - 2 434 606**
**US - A - 2 547 686**
**US - A - 2 623 882**
**US - A - 2 698 337**
**US - A - 3 644 468**

(73) Proprietor: **STAMICARBON B.V.**
**Postbus 10**
**NL-6160 MC Geleen (NL)**

(72) Inventor: **Suverkropp, Geertrudes Herman**
**Debijestraat 7**
**NL-6164 BE Geleen (NL)**
Inventor: **Nieuwkamp, Johannes Gerardus Maria**
**Grootveldstraat 11**
**NL-6164 LV Limbricht (NL)**

(74) Representative: **Pinckaers, August René et al,**
**OCTROOIBUREAU DSM Postbus 9**
**NL-6160 MA Geleen (NL)**

Courier Press, Leamington Spa, England.

Process for the preparation of succinonitrile

Background of the invention

The present invention relates to a process for preparing succinonitrile by reacting acrylonitrile with hydrocyanic acid in the presence of an alkaline catalyst. Succinonitrile is a useful product known as a raw material for preparing pyrrolidone, which can, for instance, be converted into nylon-4.

A known process for the preparation of succinonitrile is described in U.S. patent 2,434,606, according to which the alkaline catalyst may be alkali metal and earth alkali metal hydroxides, alkali metal cyanides, alkali metal carbonates, or other alkali metal salts of weak acids as well as organic bases.

According to German Auslegeschrift 1,007,313 the use of these known catalysts involves a number of disadvantages, such as, for instance, insufficient purity of the resulting product and the difficult upgrading of the reaction mixture on a technical scale. Accordingly, other catalysts have been proposed, for example the tertiary alkali metal phosphates or the alkali metal pyrophosphates.

These phosphate catalysts, however, have as a disadvantage that upon completion of the reaction they have to be separated off by means of a rather costly operation, typically filtration, after which they first have to be activated in a complicated manner before being suitable for reuse.

According to U.S. patent 2,698,337, alkaline catalysts, such as, for instance, secondary and tertiary alkylamines, quaternary ammonium hydroxides, sodium or potassium hydroxide and sodium or potassium cyanide, can successfully be used if the reaction of hydrocyanic acid with acrylonitrile is carried out with the aid of N,N-dimethylformamide or N,N-dimethylacetamide. These amides are used to form a molecular complex with the hydrocyanic acid. The use of these amides, however, not only involves additional costs, it adversely affects the purity of the final product.

In German Offenlegungsschrift No. 2,719,867 a method is described in which the reaction of hydrocyanic acid with acrylonitrile and, for instance, triethylamine as catalyst is carried out at elevated temperature, in particular at between 90 and 100°C. In view of the volatility of the reactants at such temperatures, elevated pressure or atmospheric pressure and reflux cooling are to be applied. The application of elevated pressure, however, necessitates taking costly safety measures in view of the toxicity of the reactants, while, when applying atmospheric pressure and reflux cooling, at temperatures of between 90 and 100°C a large amount of hydrocyanid acid is evaporated and refluxed. Regrettably, this procedure substantially promotes the polymerization of this compound.

According to the present invention, succinonitrile can be prepared without the disadvantages of the above-mentioned known processes.

According to the present invention, a process is disclosed for the preparation of succinonitrile by reacting acrylonitrile with hydrocyanic acid in the presence of an alkaline catalyst, the process being characterized in that triethylamine is used as a catalyst and present in an amount of at least 2 percent by weight, calculated relative to the weight of the entire reaction mixture, and that the reaction is carried out using an excess of acrylonitrile and at a temperature between 60 and 80°C.

The importance of these and other reaction parameters are discussed below.

In the process according to the present invention the amount of triethylamine in excess of the indicated amount of 2 percent by weight may be varied within certain limits. An amount larger than, for instance, 20 percent by weight of the entire reaction mixture can, if desired, be used, but this does not bring about any noticeable advantage and simply adds to the cost of the reaction. For practical purposes, we have found that an amount of from 4 to 15 percent by weight of triethylamine, calculated relative to the entire reaction mixture, is very suitable. The amount of the excess of acrylonitrile also can be varied. An amount exceeding, for instance, 3 moles of acrylonitrile per mole of hydrocyanic acid may be used, but this is not necessary to obtain a good result. Preferably, from 1.05 to 2.5 moles of acrylonitrile are used per mole of hydrocyanic acid.

Upon completion of the reaction, the resulting reaction mixture can be separated by, for instance, fractionated distillation at reduced pressure. The acrylonitrile and triethylamine thus separated can be reused in the synthesis, as described. When using an amount of triethylamine more than the amount which can be dissolved in the reaction mixture, it is possible, upon completion of the reaction, to divide the reaction mixture into two liquid layers, then to separate off the triethylamine layer thus formed, and to subject the remaining liquid layer to fractionated distillation at reduced pressure. The use of such an amount of triethylamine in excess of that which can be dissolved in the reaction mixture is advantageous in that a constant amount of triethylamine is present in the succinonitrile phase, so that, in spite of fluctuations in the amount of triethylamine added in practical scale operation, a constant reaction rate is obtained. Naturally, it will be apparent that the triethylamine layer separated off can be reused and, in case of continuous operation, returned directly to the reactor.

The process according to the invention will now be elucidated in the following examples.

Example 1

In a double-walled reaction vessel provided with baffle plates, reflux cooler, thermometer, stirrer and feed opening, succinonitrile (80 g) was mixed with acrylonitrile (63.6 g, commercial grade) and triethylamine (8.5 g). Subsequently, the coolant in the reflux cooler was brought at $-10°C$, the stirrer was started and the mixture in the reaction vessel was heated to $70°C$ with hot water that was pumped through the double wall of the reaction vessel.

Next, from a cooled feed vessel mounted on the feed opening of the reaction vessel, liquid hydrocyanic acid (27 g) was added at a fairly constant rate, in about 30 minutes, to the well-stirred contents of the reaction vessel. During the addition of hydrocyanic acid considerable reaction heat developed. The temperature of the reaction mixture was maintained at approximately $70°C$ by pumping cooling water through the double wall of the reaction vessel. The color of the reaction mixture gradually became dark brown. During the time the hydrocyanic acid was added a very slight reflux was observed in the reflux cooler, which stopped almost immediately after the addition of the hydrocyanic acid.

After the hydrocyanic acid had been added, a sample of the reaction mixture was analyzed by gas chromatography, from which it appeared that the reaction mixture contained 88.9 percent by weight of succinonitrile and 6.21 percent by weight of acrylonitrile. Analysis of another sample (titration of cyanide ions with silver nitrate) indicated that the reaction mixture contained less than 0.1 percent by weight of hydrocyanic acid.

From these analytical results, it appeared that substantially all of the hydrocyanic acid had been consumed and that succinonitrile had been formed with an efficiency of 99.0% relative to the converted acrylonitrile.

The reaction mixture obtained was distilled at a reduced pressure of 1.6 kPa. After the volatile acrylonitrile and triethylamine had been distilled off, succinonitrile was collected as a fraction that boiled at 139—140°C. This colorless fraction crystallized in the receiving flask and consisted, according to gas chromatographic analysis, of pure succinonitrile. In the distillation flask only approximately 2 g of black residue remained, which amounted to approximately 80 percent by weight of succinonitrile.

Example 2

In the same manner and in the same apparatus as described in Example 1, a test was conducted in which succinonitrile (80 g), acrylonitrile (74.2 g) and triethylamine (5.45 g) were introduced into the reaction vessel. Next, liquid hydrocyanic acid (27 g) was added over a period of approximately 30 minutes to the mixture obtained, which was kept at approximately $70°C$ by cooling. After the addition of the hydrocyanic acid a rapidly reducing hydrocyanic acid reflux was observed in the reflux cooler for one minute.

From gas chromatographic analysis of the reaction mixture it appeared that this contained 85.2 percent by weight of succinonitrile and 11.7 percent by weight of acrylonitrile. The hydrocyanic acid content of the reaction mixture was less than 0.1 percent by weight. From this analysis it appeared that succinonitrile had been formed with an efficiency of 98.8% relative to converted acrylonitrile. By distillation of the dark-colored reaction mixture at a reduced pressure of 1.6 kPa, gas chromatographically pure, colorless succinonitrile was obtained. After this distillation only 2 g of residue remained in the distillation flask.

Example 3

In the same manner as described in Example 1 an experiment was conducted in which succinonitrile (80 g), acrylonitrile (58.3 g) and triethylamine (18.5 g) were introduced into the reaction vessel. Next, liquid hydrocyanic acid (27 g) was, over a period of about 30 minutes, fed to the mixture in the reaction vessel, which was kept at approximately $70°C$ by cooling. After approximately half of the hydrocyanic acid had been fed, demixing into two liquid phases started to occur. After the hydrocyanic acid had been added, the very slight hydrocyanic acid reflux in the reflux cooler stopped practically immediately. Gas chromatographic analysis of a sample that contained representative quantities of both liquid phases proved that this contained 86.4 percent by weight of succinonitrile and 3.3 percent by weight of acrylonitrile.

From the analysis it appeared that succinonitrile had been formed with an efficiency of 98.6% relative to converted acrylonitrile. A hydrocyanic acid analysis showed that the reaction mixture contained less than 0.1 percent by weight of hydrocyanic acid.

By distillation of the reaction mixture at a reduced pressure of 1.6 kPa, gas chromatographically pure, colorless succinonitrile was obtained.

After the distillation approximately 3 g of residue remained in the distillation flask.

Example 4

To a glass reactor with an effective volume of 1750 milliliters provided with a stirrer, a reflux cooler and a circulation line with pump and cooler for discharging the reaction heat, liquid hydrocyanic acid (720 g), acrylonitrile (1695 g) and triethylamine (122 g) were supplied per hour. By controlling the temperature of the cooler, the temperature of the reaction mixture was kept at $70°C$.

The reaction product, which was continuously discharged from the reactor, contained less than 0.1 percent by weight of hydrocyanic acid. The reaction product discharged in 3.5 hours contained 83.6 percent by weight of succinonitrile, 10.9 percent by weight of acrylonitrile and 4.7 percent by weight of triethylamine. The efficiency relative to hydrocyanic acid was found to be 99.4% and the efficiency relative to consumed acrylonitrile was 99.1%. By fractionated distillation of the reaction product succinonitrile was obtained that, according to gas chromatographic analysis, was completely pure. With this distillation 96% of the succinonitrile supplied to the distillation was recovered.

## Claims

1. A process for preparing succinonitrile by reacting acrylonitrile with hydrocyanic acid in the presence of an alkaline catalyst, characterized in that as the catalyst triethylamine is used in an amount of at least 2 percent by weight, calculated relative to the entire reaction mixture, and said reaction is conducted with an excess of acrylonitrile at a temperature between 60 and 80°C.

2. A process according to claim 1, characterized in that from 4 to 15 percent by weight of triethylamine, calculated relative to the entire reaction mixture, is used as the catalyst.

3. A process according to claim 1 or 2, characterized in that for each mole of hydrocyanic acid there are from 1.05 to 2.5 moles of acrylonitrile used.

4. A process according to any of claims 1—3, characterized in that the amount of triethylamine used is more than the amount which can be dissolved in the reaction mixutre, the resulting reaction mixture is then divided into two liquid layers, one rich in triethylamine and the other rich in succinonitrile, reusing the triethylamine layer formed and fractionally distilling the succinonitrile layer formed.

## Patentansprüche

1. Verfahren zum Herstellen von Bernsteinsäurenitril durch eine Reaktion von Acrylnitril mit Blausäure in Anwesenheit eines alkalischen Katalysators, dadurch gekennzeichnet, dass als Katalysator Triäthylamin in einer Menge von mindestens 2 Gew.-% verwendet wird, bezogen auf das gesamte Reaktionsgemisch, und die Reaktion mit einem Überschuss an Acrylnitril bei einer Temperatur zwischen 60 und 80°C durchgeführt wird.

2. Verfahren nach Anspruch 1, dadurch gekennzeichnet, dass 4 bis 15 Gew.-% Triäthylamin, bezogen auf das gesamte Reaktionsgemisch, als Katalysator benutzt werden.

3. Verfahren nach Anspruch 1 oder 2, dadurch gekennzeichnet, dass für jedes Mol Blausäure 1,05 bis 2,5 Mol Acrylnitril gebraucht werden.

4. Verfahren nach den Ansprüchen 1—3, dadurch gekennzeichnet, dass die verwendete Triäthylaminmenge mehr ist als die Menge, die im Reaktionsgemisch gelöst werden kann, und das anfallende Reaktionsgemisch dan in zwei Flüssigkeitsschichten verteilt wird, eine reich an Triäthylamin und die andere reich an Bernsteinsäurenitril, wobei die Triäthylaminschicht wieder verwendet und die Bernsteinsäurenitrilschicht fraktioniert destilliert wird.

## Revendications

1. Procédé de préparation de succinonitrile en faisant réagir de l'acrylonitrile avec de l'acide cyanhydrique en présence d'un catalyseur alcalin, caractérisé en ce qu'on utilise comme catalyseur de la triéthylamine en une quantité d'au moins 2% en poids, calculée par rapport au mélange réactionnel entier, et en ce qu'on conduit ladite réaction avec un excès d'acrylonitrile à une température comprise entre 60 et 80°C.

2. Procédé selon la revendication 1, caractérisé en ce qu'on utilise comme catalyseur de 4 à 15% en poids de triéthylamine, calculé par rapport au mélange réactionnel entier.

3. Procédé selon la revendication 1 ou la revendication 2, caractérisé en ce que pour chaque mole d'acide cyanhydrique on utilise de 1,05 à 2,5 moles d'acrylonitrile.

4. Procédé selon l'une quelconque des revendications 1 à 3, caractérisé en ce que la quantité de triéthylamine utilisée dépasse la quantité que l'on peut dissoudre dans le mélange réactionnel, en ce qu'on divise alors le mélange réactionnel obtenu en deux couches liquides, l'une riche en triéthylamine et l'autre riche en succinonitrile, en ce qu'on réutilise la couche de triéthylamine formée et en ce qu'on distille de manière fractionnée la couche succinonitrile formée.